# EUROPEAN PATENT SPECIFICATION

(11) **EP 4 272 806 B1**
(45) Date of publication and mention of the grant of the patent: **25.02.2026**
(21) Application number: 23199880.8
(22) Date of filing: 12.06.2017
(51) Int. Cl.: A61N 1/36, A61N 1/05, G16H 50/20, A61B 5/12, H04R 25/00, A61B 5/053, A61B 5/388

(54) **COCHLEA HEALTH MONITORING**
COCHLEA-GESUNDHEITSÜBERWACHUNG
SURVEILLANCE DE LA SANTÉ DE LA COCHLÉE

(30) Priority: 21.06.2016 US 201615187893
(43) Date of publication of application: 08.11.2023
(62) Divisional of application: 17814841.7
(73) Proprietor: Cochlear Limited, Macquarie University, New South Wales 2109 (AU)
(72) Inventor: HEASMAN, John Michael, Sydney (AU); CAMPBELL, Luke, Sydney (AU); O'LEARY, Stephen, Sydney (AU); PLANT, Kerrie, Sydney (AU); VERHOEVEN, Kristien Johanna Maria, Sydney (AU)
(74) Representative: Reddie & Grose LLP

(56) References cited:
- US-A1- 2012 302 859
- US-A1- 2015 051 654
- US-A1- 2016 051 819
- JONATHAN C. KOPELOVICH ET AL: "Hearing Loss After Activation of Hearing Preservation Cochlear Implants Might Be Related to Afferent Cochlear Innervation Injury :", OTOLOGY & NEUROTOLOGY : AN INTERNATIONAL FORUM FOR OTOLOGY, NEUROTOLOGY, AND SKULL BASE SURGERY, vol. 36, no. 6, 1 July 2015 (2015-07-01), US, pages 1035 - 1044, XP055656263, ISSN: 1531-7129, DOI: 10.1097/MAO.0000000000000754

## Description

### BACKGROUND

### Field of the Invention

The present invention relates generally to hearing prostheses.

### Related Art

Hearing loss, a type of sensory impairment that may be due to many different causes, is generally of two types, conductive and/or sensorineural. Conductive hearing loss occurs when the normal mechanical pathways of the outer and/or middle ear are impeded, for example, by damage to the ossicular chain or ear canal. Sensorineural hearing loss occurs when there is damage to the inner ear, or to the nerve pathways from the inner ear to the brain.

Individuals who suffer from conductive hearing loss typically have some form of residual acoustic hearing (residual hearing) because the hair cells in the cochlea are undamaged. As such, individuals suffering from conductive hearing loss typically receive an auditory prosthesis that generates motion of the cochlea fluid. Such auditory prostheses include, for example, acoustic hearing aids, bone conduction devices, electro-acoustic devices, and direct acoustic stimulators.

In many people who are profoundly deaf, however, the reason for their deafness is sensorineural hearing loss. Those suffering from some forms of sensorineural hearing loss are unable to derive suitable benefit from auditory prostheses that generate mechanical motion of the cochlea fluid. Such individuals can benefit from implantable auditory prostheses that stimulate nerve cells of the recipient's auditory system in other ways (e.g., electrical, optical and the like). Electro-acoustic devices are often proposed when there is residual acoustic hearing in, for example, the lower frequency ranges that can be utilized for sound perception (e.g., via conventional acoustic amplification), and sound perception can be evoked in the higher frequencies via a cochlear implant.. An auditory brainstem stimulator is another type of stimulating auditory prosthesis that might also be proposed when a recipient experiences sensorineural hearing loss due to damage to the auditory nerve.

D1 - US2015051654A1 relates to a system including a programming device configured to be located external to a cochlear implant, a programming interface device that is communicatively coupled to the programming device and configured to be located external to the patient, and a receiver communicatively coupled directly to the programming interface device. The programming device directs at least one of the cochlear implant system and the receiver to apply a stimulation to the patient, then records an evoked response that occurs in response to the stimulation, and performs a predetermined action in response to the evoked response (*Abstract of D1*). For instance, to evaluate the residual hearing status of the patient, programming device 120 may direct receiver 124 to apply acoustic stimulation to the patient and then determine whether an evoked response occurs in response to the acoustic stimulation. If an evoked response does occur, the programming device 120 may evaluate the residual hearing of the patient by comparing the evoked response to a previously recorded evoked response and determine based on a comparison that the patients residual hearing is changing *(Paragraph 0064-0066 of D1*).

### SUMMARY OF THE INVENTION

According to the invention, there is provided a hearing prosthesis according to claim 1, advantageous implementations being specified in the dependent claims.

### SUMMARY OF THE DISCLOSURE

In one aspect, a method is provided. The method comprises: obtaining, at a hearing prosthesis, one or more biomarkers each associated with a cochlea health of a recipient of the hearing prosthesis; analyzing, at the hearing prosthesis, the one or more biomarkers to identify one or more precursors of a change to the cochlea health; and in response to identification of at least one precursor of a change to the cochlea health, initiating one or more remedial actions at the hearing prosthesis.

In another aspect, a hearing prosthesis is provided. The hearing prosthesis comprises: a stimulating assembly configured to be implanted in a recipient's cochlea, wherein the stimulating assembly comprises a plurality of stimulating contacts configured to deliver electrical stimulation signals to the cochlea; and a processor configured to: predict, based on one or more cochlea health biomarkers, future residual hearing loss of the recipient's cochlea; and responsive to the prediction of the residual hearing loss, adjust operation of the c hearing prosthesis to remediate the future residual hearing loss.

In yet another aspect, a method is provided. The method comprises: obtaining one or more biomarkers each associated with residual hearing of a recipient of a hearing prosthesis; and employing the one or more biomarkers as real-time feedback for regulation of stimulation signals which originate from the hearing prosthesis to provide for the long-term preservation of the residual hearing.

### BRIEF DESCRIPTION OF THE DRAWINGS

Embodiments are described herein in conjunction with the accompanying drawings, in which:
FIG. 1A is a schematic diagram of an electro-acoustic hearing prosthesis in accordance with embodiments presented herein;
FIG. 1B is a block diagram of the electro-acoustic hearing prosthesis of FIG. 1A;
FIG. 1C is a schematic diagram illustrating of the electro-acoustic hearing prosthesis of FIG. 1A;
FIG. 2 is a diagram illustrating cochlea health analysis techniques in accordance with embodiments presented herein;
FIG. 3 is a block diagram of a cochlea health analysis module in accordance with embodiments presented herein; and
FIG. 4 is a flowchart of a method in accordance with embodiments presented herein.

### DETAILED DESCRIPTION

Auditory/hearing prosthesis recipients suffer from different types of hearing loss (e.g., conductive and/or sensorineural) and/or different degrees/severity of hearing loss. However, it is now common for many hearing prosthesis recipients to retain some residual natural hearing ability (residual hearing) after receiving the hearing prosthesis. That is, hearing prosthesis recipients often retain at least some of their natural ability to hear sounds without the aid of their hearing prosthesis. There has been increased focus on preserving a recipient's hearing during implantation of a hearing prosthesis into a recipient. For example, in the case of cochlear implants, progressive improvements in the design of intra-cochlear electrode arrays (stimulating assemblies), surgical implantation techniques, tooling, *etc.* have enabled atraumatic surgeries which preserve at least some of the recipient's fine inner ear structures (e.g., cochlea hair cells) and the natural cochlea function, particularly in the higher frequency regions of the cochlea. Accordingly, greater numbers of recipients have post-implantation residual hearing in an ipsilateral and/or contralateral ear. The benefits of post-implantation residual hearing may include, for example, improved sound localization, music appreciation, binaural release from unmasking, head shadow, and the ability to distinguish acoustic signals in a noisy environment.

However, there is also risk that a recipient's residual hearing, and more generally a recipient's cochlea health, can change or deteriorate post-implantation (i.e., after the hearing prosthesis has been implanted within the recipient). These changes may be related to, for example, inner ear inflammatory responses, fibrosis, ossification, auditory neuropathy, endolymphatic hydrops, electro-acoustic interaction/masking, and excitotoxicity. For example, a hearing prosthesis that is improperly configured for (i.e., improperly "fit" to) a recipient may result in the delivery of stimulation (electrical stimulation and/or acoustic stimulation) in a manner that causes acute and/or chronic losses in the recipient's residual hearing and/or in a manner that interferes with the transduction of information via the residual hearing structures.

In certain cases, losses to a recipient's residual hearing could render the recipient's hearing prostheses ineffective. In other cases, continued operation of the hearing prostheses could exacerbate the residual hearing loss. Presently, residual hearing loss and other changes to a recipient's cochlea health are only detected/identified within a clinical environment, typically using complex equipment and techniques implemented by trained audiologists/clinicians. However, recipients generally do not visit clinics on a regular basis due to, for example, costs, low availability of trained audiologists, such as in rural areas, *etc.* Therefore, the need to visit a clinic in order to residual hearing and/or other cochlea health changes may not only be cost prohibitive for certain recipients, but may also result in the passage of a significant period of time before the residual hearing or other cochlea health change is identified, let alone addressed. Accordingly, conventional techniques are designed to detect residual hearing and/or other cochlea health changes only after such changes have occurred. That is, conventional techniques can only operate to retroactively address residual hearing and/or other cochlea health changes.

Presented herein are in-situ techniques for monitoring a recipient's cochlea health to proactively identify (i.e., predict) changes to the recipient's cochlea health outside of a clinical setting. As used herein, changes to a recipient's "cochlea health" includes changes in the recipient's residual hearing, such as changes to the function of the cochlea hair cells, synapses, spiral ganglions, dendrites, peripheral central processes, as well as other changes in the functioning of the cochlea/ inner ear. As described further below, the cochlea health monitoring techniques presented herein obtain one or more cochlea health biomarkers associated with a recipient's cochlea health, such as the recipient's residual hearing, and analyze these biomarkers to predict that a cochlea health change, sometimes referred to herein as an "inner ear crises," is likely to occur. Also as described further below, the techniques presented herein can be carried out repeatedly with minimal or no involvement, or possibly even awareness, by the recipient, and may be used to proactively remediate (e.g., prevent) changes to a recipient's cochlea health (e.g., ensure the stimulus is safe and to ensure on-going clinical utility).

For ease of illustration, embodiments are primarily described herein with reference to one type of implantable auditory/hearing prosthesis, namely an implantable electro-acoustic hearing prosthesis that is configured to deliver both electrical stimulation (i.e., electrical stimulation signals) and acoustic stimulation (i.e., acoustic stimulation signals) to a recipient. Acoustic stimulation combined with electrical stimulation is sometimes referred to herein as electro-acoustic stimulation. However, it is to be appreciated that the techniques presented herein may be used with other types of hearing prostheses, such as cochlear implants, auditory brainstem stimulators, bimodal hearing prostheses direct acoustic stimulators, bone conduction devices, *etc.* It is also to be appreciated that the techniques presented herein may be used with electro-acoustic hearing prostheses comprising different types of output devices (e.g., receivers, direct acoustic stimulators, bone conduction devices, intra-cochlear stimulating assemblies, auditory brain implants, *etc.).*

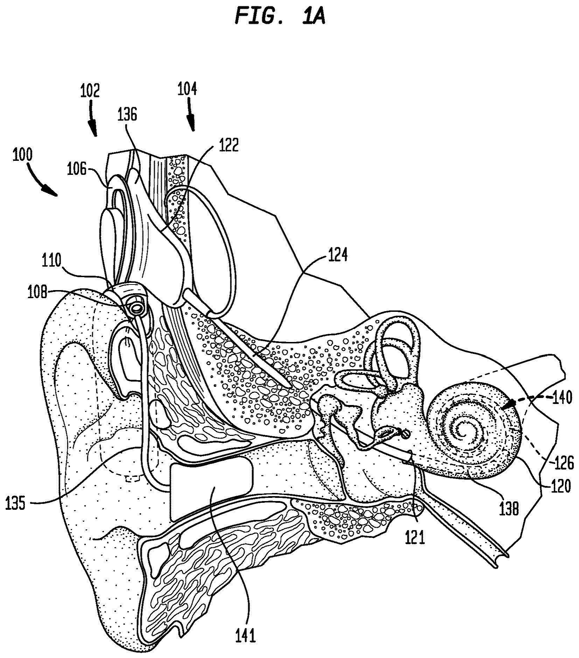

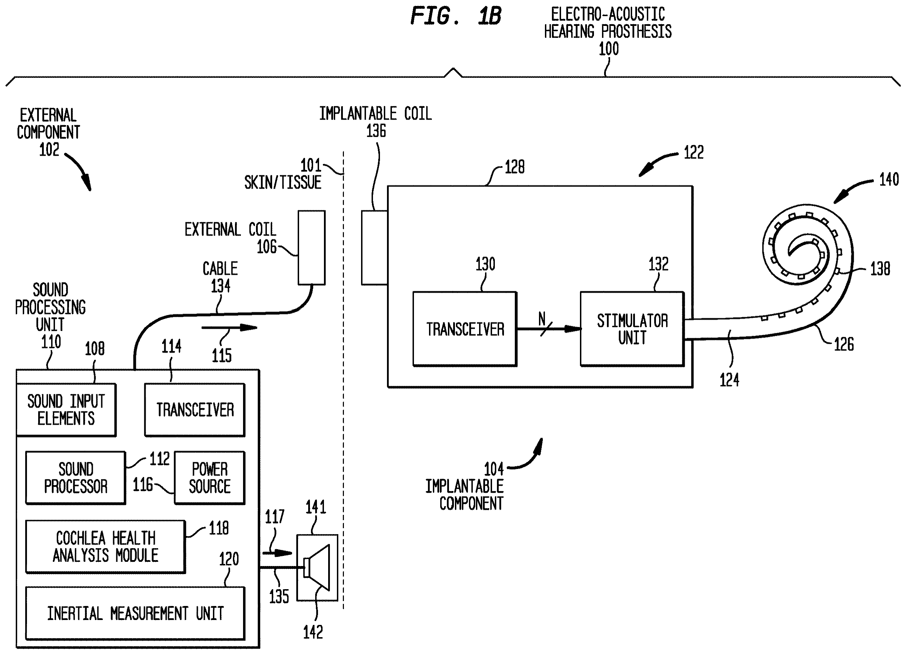

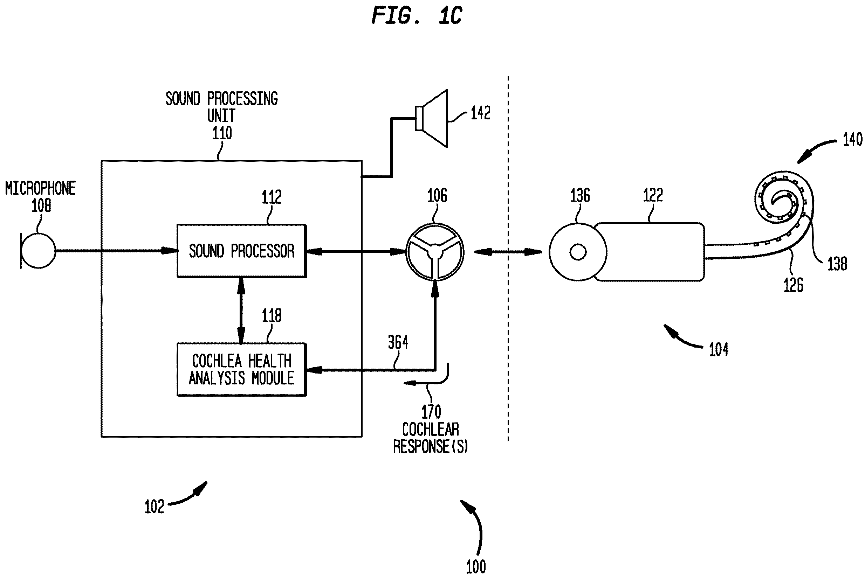

FIGs. 1A, 1B, and 1C are diagrams of an illustrative implantable electro-acoustic hearing prosthesis configured to implement the cochlea health monitoring techniques presented herein. More specifically, FIGs. 1A and 1B illustrate an electro-acoustic hearing prosthesis 100 that includes an external component 102 and an internal/implantable component 104. The external component 102 is configured to be directly or indirectly attached to the body of a recipient, while the implantable component 104 is configured to be subcutaneously implanted within the recipient (i.e., under the skin/tissue 101 of the recipient). FIG. 1C is a schematic diagram illustrating further details of the external component 102, namely operation of the cochlea health analysis module 118.

The external component 102 comprises an external coil 106 and a sound processing unit 110 connected via, for example, a cable 134. The external coil 106 is typically a wire antenna coil comprised of multiple turns of electrically insulated single-strand or multi-strand platinum or gold wire. Generally, a magnet (not shown in FIG. 1A) is fixed relative to the external coil 106. The external component 102 also comprises a hearing aid component 141 that includes a receiver 142 (FIG. 1B). The hearing aid component 141 is connected to the sound processing unit 110 via a cable 135. The receiver 142 is a component that is configured to deliver an acoustic signal (acoustic stimulation) to the recipient via the recipient's ear canal and middle ear. The receiver 142 may be, for example, positioned in or near the recipient's outer ear.

The sound processing unit 110 comprises one or more sound input elements, such as microphones 108, a sound processor 112, an external transceiver unit (transceiver) 114, a power source 116, a cochlea health analysis module 118, and an inertial measurement unit ("IMU") 120. The sound processing unit 110 may be, for example, a behind-the-ear ("BTE") sound processing unit or other type of processing unit worn on the recipient's head.

The implantable component 104 comprises an implant body (main module) 122, a lead region 124, and an elongate intra-cochlear stimulating assembly (electrode array) 126. The implant body 122 generally comprises a hermetically-sealed housing 128 in which an internal transceiver unit (transceiver) 130 and a stimulator unit 132 are disposed. The implant body 122 also includes an internal/implantable coil 136 that is generally external to the housing 128, but which is connected to the transceiver 130 via a hermetic feedthrough (not shown). Implantable coil 136 is typically a wire antenna coil comprised of multiple turns of electrically insulated single-strand or multi-strand platinum or gold wire. The electrical insulation of implantable coil 136 is provided by a flexible molding (e.g., silicone molding), which is not shown in FIG. 1B. Generally, a magnet (not shown in FIG. 1B) is fixed relative to the implantable coil 136.

Elongate stimulating assembly 126 is configured to be at least partially implanted in the recipient's cochlea (not shown in FIG. 1B) and includes a plurality of longitudinally spaced intra-cochlear stimulating contacts (e.g., electrical and/or optical contacts) 138 that collectively form a contact array 140. Stimulating assembly 126 extends through an opening in the cochlea (e.g., cochleostomy, the round window, *etc.)* and has a proximal end connected to stimulator unit 132 via lead region 124 and a hermetic feedthrough (not shown in FIG. 1B). As such, lead region 124 couples the stimulating assembly 126 to the implant body 122 and, more particularly, stimulator unit 132.

Returning to external component 102, the microphone(s) 108 and/or other sound input elements (not shown) are configured to detect/receive sound signals and generate electrical output signals therefrom. These output signals are representative of the detected sound signals. In addition to the one or more microphones 108, the sound processing unit 110 may include other types of sound input elements (e.g., telecoils, audio inputs, *etc.)* to receive sound signals. However, merely for ease of illustration, these other types of sound input elements have been omitted from FIG. 1B.

The sound processing unit 110 includes the inertial measurement unit 120. The inertial measurement unit 120 is configured to measure the inertia of the recipient's head, that is, motion of the recipient's head. As such, inertial measurement unit 120 comprises one or more sensors 125 each configured to sense one or more of rectilinear or rotatory motion in the same or different axes. Examples of sensors 125 that may be used as part of inertial measurement unit 120 include accelerometers, gyroscopes, compasses, and the like. Such sensors may be implemented in, for example, micro electromechanical systems (MEMS) or with other technology suitable for the particular application.

The sound processor 112 is configured execute sound processing and coding to convert the output signals received from the sound input elements into coded data signals that represent acoustic and/or electrical stimulation for delivery to the recipient. That is, as noted, the electro-acoustic hearing prosthesis 100 operates to evoke perception by the recipient of sound signals received by the sound input elements (e.g., microphones 108) through the delivery of one or both of electrical stimulation signals and acoustic stimulation signals to the recipient. As such, depending on a variety of factors, the sound processor 112 is configured to convert the output signals received from the sound input elements into a first set of coded signals representative of electrical stimulation and/or into a second set of coded signals representative of acoustic stimulation. The coded signals representative of electrical stimulation are represented in FIG. 1B by arrow 115, while the coded signals representative of acoustic stimulation are represented in FIG. 1B by arrow 117.

The coded signals 115 are provided to the transceiver 114. The transceiver 114 is configured to transcutaneously transfer the coded signals 115 to the implantable component 104 via external coil 106. More specifically, the magnets fixed relative to the external coil 106 and the implantable coil 136 facilitate the operational alignment of the external coil 106 with the implantable coil 136. This operational alignment of the coils enables the external coil 106 to transmit the coded data signals 115, as well as power signals received from power source 116, to the implantable coil 136. In certain examples, external coil 106 transmits the signals to implantable coil 136 via a radio frequency (RF) link. However, various other types of energy transfer, such as infrared (IR), electromagnetic, capacitive and inductive transfer, may be used to transfer the power and/or data from an external component to an electro-acoustic hearing prosthesis and, as such, FIG. 1B illustrates only one example arrangement.

In general, the coded data and power signals are received at the transceiver 130 and provided to the stimulator unit 132. The stimulator unit 132 is configured to utilize the coded data signals 115 to generate stimulation signals (e.g., current signals) for delivery to the recipient's cochlea via one or more stimulating contacts 138. In this way, electro-acoustic hearing prosthesis 100 stimulates the recipient's auditory nerve cells, bypassing absent or defective hair cells that normally transduce acoustic vibrations into neural activity, in a manner that causes the recipient to perceive the received sound signals.

As noted above, it is common for hearing prosthesis recipients to retain at least part of this normal hearing functionality (i.e., retain at least one residual hearing). Therefore, the cochlea of a hearing prosthesis recipient can be acoustically stimulated upon delivery of a sound signal to the recipient's outer ear, with the aid of the hearing prosthesis itself or, in certain cases, without the aid of the hearing prosthesis. In the example of FIGs. 1A and 1B, the receiver 142 is used to aid the recipient's residual hearing. More specifically, the coded signals 117 (i.e., the signals representative of acoustic stimulation) are provided to the receiver 142. The receiver 142 is configured to utilize the coded signals 117 to generate the acoustic stimulation signals that are provided to the recipient. In other words, the receiver 142 is used to enhance, and/or amplify a sound signal which is delivered to the cochlea via the middle ear bones and oval window, thereby creating dynamic pressure changes in the perilymph within the cochlea.

As noted above, a recipient's cochlea health (e.g., residual hearing, neural survival, *etc.),* can change post-implantation. Therefore, in accordance with embodiments presented herein, the electro-acoustic hearing prosthesis 100 comprises the cochlea health analysis module 118 that is configured to proactively identify (i.e., predict) post-implantation changes to a recipient's cochlea health and, potentially, initiate one or more remedial actions to, for example, prevent significant changes to the recipient's cochlea health. More specifically, the cochlea health analysis module 118 is configured to measure, determine, or otherwise obtain one or more biomarkers associated with the recipient's cochlea health. The cochlea health analysis module 118 is then configured to analyze these biomarkers to detect/identify one or more precursors of a change to the cochlea health (i.e., inner ear crises), such as precursors to residual hearing loss. As used herein, "precursors" to cochlea health changes are detectable events corresponding to patterns of behavior or established patterns relating to known pathologies, pathophysiology or physiology that correlate to potential future changes in the cochlea health, such as potential loss of residual hearing (i.e., predetermined risk factors for residual hearing loss) and or trauma to inner ear biological structures.

In one form, the precursors to cochlea health are electro-acoustic phenomena within the recipient's cochlea that are suggestive of potential residual hearing loss (e.g., significant residual hearing loss, residual hearing loss that is noticeable/perceivable by the recipient, *etc.).* As used herein, "electro-acoustic phenomena" are interactions of electrical and acoustic stimulation, or effects thereof, that can be detected and correlated to patterns of behavior or established patterns relating to known pathologies, pathophysiology or physiology associated with residual hearing loss.

Based on the analysis of the biomarkers and the identification of one or more cochlea health change precursors, the cochlea health analysis module 118 can initiate one or more actions (treatments) to remediate the cochlea health changes. In one example, the cochlea health analysis module 118 operates as a closed-loop element that is configured to auto-regulate, for example, the delivered stimulation (acoustic and electric stimulation signal properties/parameters) in order to minimize the likelihood of damaging or masking the physiological structures supporting residual hearing (e.g., a real-time control / regulatory system to minimize the loss of residual hearing post-implantation). Without regulation of the delivered stimulation, there is a risk that the post-implantation residual hearing may be compromised by the inadequately configured stimulation. For example, either the electrical and acoustic stimulation in combination (electro-acoustic) or in isolation (electric or acoustic) could be improperly clinically configured so as to cause either acute or chronic changes (injurious) to the cochlear health or, to interfere with the transduction of information via the residual acoustic hearing structures (e.g., by electroacoustic interactions including masking).

The closed-loop features of the embodiments presented herein allow a hearing prosthesis to constantly adapt and update stimulation based on the measured biomarkers. This feature can be used to efficiently respond to, for example, a transient change to the cochlea due to an acute inflammatory response, or a chronic inflammatory process that may potentially induce permanent loss of hearing or progressive loss in residual hearing. That is, a hearing prosthesis in accordance with embodiments presented herein may employ the biomarkers as real-time feedback for regulation of the stimulation signals which originate from the hearing prosthesis to provide for the long-term preservation of the recipient's cochlea health, including residual hearing.

FIGs. 1A-1C illustrate one example arrangement for the electro-acoustic hearing prosthesis 100. However, it is to be appreciated that embodiments of the present invention may be implemented in electro-acoustic devices having alternative arrangements and/or in other types of hearing prostheses.

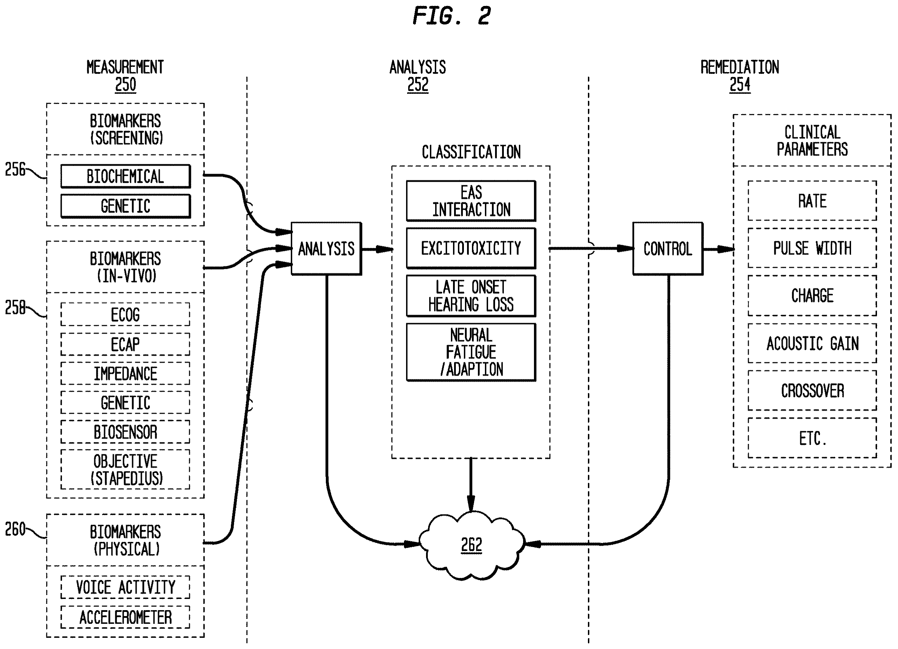

FIG. 2 is a schematic diagram illustrating techniques presented herein for in-situ proactive/predictive identification of cochlea health changes, for example, in the form of residual hearing loss by a recipient. The technique of FIG. 2 decreases the likelihood that the recipient of an electro-acoustic hearing prosthesis will develop a perceivable residual hearing loss (i.e., a residual hearing loss that is perceivable by the recipient). For ease of illustration, the example of FIG. 2 will be described with reference to the electro-acoustic hearing prosthesis 100 shown in FIGs. 1A-1C.

Shown in FIG. 2 are three (3) general sections/stages, referred to as a measurement stage 250, an analysis stage 252, and a remediation stage 254. In the measurement stage 250, the electro-acoustic hearing prosthesis 100, more specifically cochlea health analysis module 118, is configured to measure or otherwise determine/obtain one or more cochlea health biomarkers. As shown in FIG. 2, there are at least three different types of biomarkers that may be obtained by the cochlea health analysis module 118, including external or screening biomarkers 256, in-vivo biomarkers 258, and physical biomarkers 260. Screening biomarkers 256 are pre-determined biomarkers obtained through external processes, such as biochemical analyses, genetic screening, medical health records, and factors relating to hearing loss such as etiology and duration of deafness *etc.* These pre-determined biomarkers can be employed within analytical models to optimize the run time system. Here, the analytical model can, for example, segment or categorize the recipient based on established normative data. These categories can then decide the type of predictions and remedial actions may be taken by the live system (i.e., the hearing prosthesis operating in real-time). These remedial actions may, in certain arrangements, be tailored for high risk groups who are at a heightened danger of losing their residual hearing post operatively.

The physical biomarkers 260 are biomarkers that relate to one or more physical attributes. These physical attributes can include, for example, physical movements (e.g., detected by inertial measurement unit 120), sound parameters (e.g., voice activity detections determined by sound processor 112), *etc.* Distinct from the pre-determined biomarkers collected by medical health records, the physical attributes are inputs from the hearing prosthesis that are unrelated to physiological measurements from the cochlea or auditory pathway. Instead, the physical biomarkers 260 are associated with the recipient's movements, such as head movement and ambulatory motions. Some of the physical biomarkers 260 provide information characterizing, over time, the patients stability on their feet and head movements, Changes or particular patterns (pre-determined) of these physical attributes can provide predictions on whether the patient may be experiencing an onset or episode of vertigo or dizziness. These physical biomarkers 260 may be employed either alone or in conjunction with other biomarkers to predict the onset of an inner ear crisis.

Other physical biomarkers 260 may include monitored attributes of the recipient's own voice. Such monitoring can provide details on the vocal production of the individual, such as duration of utterance and conversational turns. As per the physical characterization, such information may yield, over time, changes to the recipient's hearing. For example, changes to the voiced production may indicate a change to the peripheral auditory pathway of the individual, reflecting a change to the inner ear and function. Again, these features may be either used independent or in conjunction with other inputs for automatic assessment of cochlea health.

The in-vivo biomarkers 258 are biomarkers obtained by the cochlea health analysis module 118 through one or more in-situ measurements, such as cochlear response telemetry measurements, electrophysiological measurements, and biochemical measurements. Cochlear response telemetry measurements include electrocochleography (ECoG) measurements, electrically evoked compound action potential (ECAP) measurements, higher evoked potentials measured from the brainstem and auditory cortex, and measurements of the electrical properties of the cochlea and the electrode array. Measurements of the electrical properties of the cochlea and the electrode array are generally and collectively referred to herein as "cochlea potential" measurements, and may include a number of voltage measurements, such as impedance measurements.

In addition, the hearing prosthesis may receive inputs from sensors, sometimes referred to herein as biosensors, that are capable of extracting information relating to the homeostasis of the inner ear. The homeostasis of the inner ear refers to the constant equilibrium of the chemical environment in the inner ear. Maintenance of the homeostasis is critical to ensure the cells related to hearing function are protected and maintained. Examples of the types of biochemical feedback from these sensors might be the level of potassium and sodium levels in the perilymph in which the electrode array is immersed. Other chemical and biochemical measures may be taken to ascertain if homeostasis has been compromised or has altered since implantation of the cochlear implant.

Electrophysiological measurements and electrical properties refer to the use of the signals that are of biological origin or relate to the biological properties (electric potentials) as inputs to the analysis stage of the real-time algorithm. Each recipient may have, based on the pre-determined inputs, normal patterns or characteristic behaviors for each of these electrophysiological and cochlea potentials. These signals may be measured continuously during operation of the system and compared against these normative templates or patterns of normal behavior in an effort to determine whether the inner ear has altered in its health or function. Alternatively, individual or multiple inputs may be compared against each other or against prior time points to determine a similar analysis. The cochlea potentials may change in response to physiological changes that are independent from implant function, or as a direct consequence to either the acoustic and or electrical stimulus of the cochlea by the implant system.

An ECAP measurement refers to the capture of a set of electrical potentials generated in the recipient's cochlea 120 in response to the delivery of electrical stimulation to the cochlea. In contrast, an ECoG measurement refers to the capture of a set of electrical potentials generated in the recipient's cochlea 120 in response to the delivery of acoustic stimulation to the cochlea. The captured electrical potentials (i.e., a set of ECoG responses) may include a plurality of different stimulus related electrical potentials, such as the cochlear microphonic (CM), the cochlear summating potential (SP), the auditory nerve neurophonic (ANN), and the auditory nerve Action Potential (AP), which are measured independently or in various combinations. The cochlear microphonic is an alternating current (AC) voltage that mirrors the waveform of the acoustic stimulus at both low, moderate and high levels of acoustic stimulation. The cochlear microphonic is generated by the outer hair cells of the organ of Corti and is dependent on the proximity of the recording electrode(s) to the stimulated hair cells and the basilar membrane. In general, the cochlear microphonic is proportional to the displacement of the basilar membrane by the travelling wave phenomena.

The summating potential is the direct current (DC) response of the outer hair cells of the organ of Corti as they move in conjunction with the basilar membrane (i.e., reflects the time-displacement pattern of the cochlear partition in response to the stimulus envelope). The summating potential is the stimulus-related potential of the cochlea and can be seen as a DC (unidirectional) shift in the cochlear microphonic baseline. The direction of this shift (i.e., positive or negative) is dependent on a complex interaction between stimulus parameters and the location of the recording electrode(s).

The auditory nerve neurophonic is a signal recorded from the auditory nerve, while the auditory nerve Action Potential represents the summed response of the synchronous firing of the nerve fibers in response to the acoustic stimuli, and it appears as an alternating current voltage. The auditory nerve Action Potential is characterized by a series of brief, predominantly negative peaks, including a first negative peak (N1) and second negative peak (N2). The auditory nerve Action Potential also includes a magnitude and a latency. The magnitude of the auditory nerve Action Potential reflects the number of fibers that are firing, while the latency of the auditory nerve Action Potential is measured as the time between the onset and the first negative peak (N1).

In the example of FIGs. 1A-1C, an ECoG measurement and/or an ECAP measurement is initiated by the cochlea health analysis module 118. In the case of an ECoG measurement, the cochlea health analysis module 118 is configured to cause the receiver 142 to deliver acoustic stimulation to the recipient's cochlea 120 while, in the case of an ECAP measurement, the cochlea health analysis module 118 is configured to cause the stimulator unit 132 to deliver electrical stimulation to the recipient's cochlea 120. Following delivery of the acoustic or electrical stimulation, the cochlea analysis health module 118 is configured to receive a group/set of cochlear responses 170 (FIG. 1C). As noted, the cochlear responses 170 (i.e., the ECoG or ECAP responses) are electrical potentials generated in the recipient's cochlea 120 when acoustic or electric stimulation are delivered to the cochlea 120. The cochlear responses 170 are obtained by one or more of the intra-cochlea electrodes 138 and are transmitted through the external coil 106 to the sound processor 112 and/or the cochlea health analysis module 118. In an embodiment, the cochlear responses 170 are transmitted from the external coil 106, through the sound processor 112, to the cochlea health analysis module 118. In another embodiment, the cochlear responses 370 are transmitted from the external coil 106 directly to the cochlea health analysis module 118.

The ECoG measurements and/or an ECAP measurements may be performed, for example, periodically, at preselected times, in response to user inputs, *etc.* In certain examples, the recipient may be provided with a notification indicating that measurements are about to be performed. Alternatively, the measurements may be conducted at sub-clinical levels that cannot be perceived by the user. The measurements may also be inter-dispersed in the clinical operation of the device such that the measures are not perceived and can be obtained continuously.

In accordance with certain embodiments presented herein, a hearing prosthesis is configured to analyze ambient sound signals received by the hearing prosthesis during normal operation (i.e., outside of a clinical setting) to identify portions of the sound signals that are sufficient to evoke an ECoG response from the cochlea. When a portion of a sound signal that is sufficient to evoke an ECoG response is identified, the hearing prosthesis itself performs an ECoG measurement using one or more implanted electrodes.

As noted, FIGs. 1A, 1B, and FIG. 2 illustrate the use of a receiver 142 to deliver acoustic stimulation for the performance of an ECoG measurement. However, embodiments of the present invention may be implemented in other hearing prostheses that deliver stimulation in a different manner to evoke an ECoG measurement (e.g., bone conduction devices or direct acoustic stimulators that deliver vibration to the cause movement of the cochlea fluid). It is also to be appreciated that embodiments of the present invention may be implemented in hearing prostheses that do not deliver acoustic stimulation.

More specifically, as noted above, it is common for hearing prosthesis recipient's to retain at least part of this normal hearing functionality (i.e., retain at least one residual hearing). Therefore, the cochlea of some hearing prosthesis recipient can be acoustically stimulated upon delivery of a sound signal to the recipient's outer ear without the aid of the hearing prosthesis itself (e.g., recipient's who are implanted with only a cochlear implant). In such recipients, an ECoG measurement may be performed in response to the un-aided acoustic stimulation.

Returning to the specific example of FIG. 2, the analysis stage 252 involves the analysis and/or classification of the cochlea health biomarkers obtained during the measurement stage 250. In particular, the cochlear health analysis module 118 is configured to employ the real-time analysis of the cochlea health biomarkers (e.g., in-situ and/or external inputs) to determine, again in real-time, whether the recipient is likely to develop a perceivable residual hearing loss at some point in the future. Stated differently, the biomarkers are analyzed by a real-time algorithm to identify precursors to cochlea health changes (i.e., inner ear crises), such as future residual hearing loss (e.g., significant residual hearing loss, residual hearing loss that is noticeable/perceivable by the recipient, *etc.).*

The analysis of the various biomarkers to identify precursors to cochlea health changes may take a number of different forms. In one embodiment, the biomarkers are used to identify detectable events corresponding to patterns of behavior or established patterns relating to known pathologies, pathophysiology or physiology that correlate to potential future changes in the cochlea health, such as potential loss of residual hearing (i.e., predetermined risk factors for residual hearing loss) and or trauma to inner ear biological structures. Stated differently, the biomarkers may be used as inputs to a pattern matching algorithm that correlates various combinations of the biomarkers with patterns of behavior or established patterns relating to known pathologies, pathophysiology or physiology that correlate to inner ear crises. For example, the biomarkers may be analyzed to identify electro-acoustic phenomena within the recipient's cochlea that are suggestive of a cochlea health change, such as a potential residual hearing loss, and to classify the type or cause of the expected residual hearing loss. That is, the analysis of the morphology of various signals, possibly relative to each other, can identify/reveal events and/or patterns that occur even before a recipient's hearing changes (i.e., precursors to residual hearing changes). For example, an ECoG biomarker detected during measurement stage 250 may indicate that the stimulus input is at risk of causing excitotoxicity, which might contribute to either an acute or permanent loss of inner ear function that may manifest as a perceivable or non-perceivable residual hearing loss in the future. In that instance, the ECoG biomarker would be classified as related to excitotoxicity.

It is to be appreciated that the real-time algorithm may classify any number of biomarkers with different causes of perceivable residual hearing loss. For example, an ECoG biomarker may be classified as related to excitotoxicity, as well as to late onset hearing loss. In another example, an ECoG biomarker and an ECAP biomarker may both be classified as related to excitotoxicity.

FIG. 2 illustrates several possible "classifications" or "categories" of potential causes of perceivable residual hearing loss in recipients of electro-acoustic hearing prostheses for different biomarkers that may be determined and used by the cochlea health analysis module (i.e., the analysis state of the real-time algorithm executed by the hearing prosthesis). These classifications, which are further described below, include: (1) electroacoustic interaction, (2) excitotoxicity, (3) late onset hearing loss, and (4) neural fatigue or adaption. One skilled in the art will recognize that the causes described below are merely examples and that there are many causes and/or effects of perceivable residual hearing loss other than those provided herein. For example, in another embodiment the biomarkers may be used to classify dizziness or vertigo, which may be a result of residual hearing loss. In certain examples, the classifications may be utilized to "grade" the severity of the predicted hearing loss (e.g., low, medium, high). The assigned severity grade may be used to, for example, select the type of remedial action that is initiated, the timing of the remedial action, etc.

Referring first to electro-acoustic interaction, the concurrent provision of electrical and acoustic stimulation to the cochlea, be it aided or un-aided, can impair the transduction of an effective audibility of the residual acoustic hearing. This impairment relates to a reduction in quality of information (e.g., the magnitude and/or timing of a signal, *etc.)* presented to the acoustic structures. This particular impairment is driven by the interaction of the electrical stimulus via electrophonic effects or via recruitment of the neural structures through the spread of excitation. Either or both types of interaction may occur due to the wide spread of the electrical field associated with the delivery of electrical stimulus in commercial cochlear implant systems. The clinical impact of such interaction can include uncontrolled loudness, loss of timing information (e.g., localization or hearing in ambient noise), off-frequency hearing, and informational and energetic masking of information. Any of these impacts can lead to a loss in perceivable residual hearing (e.g., poorer clinical outcomes for the recipient).

Referring next to excitotoxicity, systems capable of delivering large amounts of charge, chronically, in the presence of either aided or un-aided acoustic hearing may pose a risk, either directly or indirectly, to the residual hearing or the overall cochlea health. In particular, the large spread of an applied electrical field and charge associated with electrical stimulation of the cochlea may chronically hyperpolarize the pre-synaptic acoustic hearing structures (including the synapse). If electrical stimulation is delivered chronically, this hyperpolarization may cause temporary or permanent damage to neural pathways, thereby causing a loss in perceivable residual hearing and or electrical hearing for the user.

Referring next to late onset hearing loss, following the surgical insertion of an electrode array into the cochlea, the structure is prone to additional physical, biological, acoustic, and/or electrical trauma. That is, a recipient's inner ear undergoes a number of significant mechanical, biological and biochemical events during surgical implantation, recovery, and during initiation and management of the clinical settings of the electro-acoustic hearing prosthesis. In the case of acoustic and electrical stimulation applied to the cochlea, there is a practical risk that the clinical stimulus, if set excessively, may trigger an immunological response from the cochlea through either direct or indirect mechanisms. Such an immunological response may lead to transient changes in the electrode impedance, corresponding changes in the vestibular system, and hydrops, *etc.* These changes may manifest clinically as dizziness, out-of-compliance states, fluctuating hearing levels, and temporary or permanent loss of residual hearing.

Referring next to neural fatigue/adaption, excessive delivery of electro-acoustic stimulation has been demonstrated to induce neural fatigue or long-term adaption in the neurophysiological structures of the cochlea. The onset of either or both of these phenomena may require device configuration changes or else impact the electro-acoustic hearing prosthesis if incoming stimuli are set at excessive levels. Neural fatigue here refers to either partial or complete conduction block of the neural signals evoked due to either acoustic or electrical stimulation, or a combination thereof. This blocking relates to the neurological mechanism of the nerve in that signals evoked at the periphery of the auditory nerve are either partially or fully blocked further up the pathway. In contrast, neural adaption relates to the innate regulatory system of the neurons whereby the size of the evoked response decreases or increases based on the type and duration of the stimulus input. For example, a prolonged stimulus input to the cochlea may decrease in perceived loudness due to the user. Both neural fatigue and adaption may be characterized for the user under normal operating conditions for the cochlear implant and then compared against prior time-points or patterns of pre-determined behavior associated with diagnosed cochlea health changes.

Furthermore, regarding dizziness/vertigo, it has been long established that recipients of cochlear implants may present with mild to severe episodes of dizziness or vertigo following implantation. The exact cause (pathology) of this remains somewhat unclear however it is thought, in some cases, to be a secondary effect of an underlying disruption to increased pressure within the endolymphatic space or an acute or chronic inflammatory response within the inner ear.

As noted, in the analysis stage 252, the cochlea health analysis module 118 analyzes one or more of the above biomarkers in real-time to predict, and potentially classify, a recipient's cochlea health change, such as residual hearing loss. In certain embodiments, the cochlea health analysis module 118 is configured to collectively analyze a plurality of different objective inputs to predict changes to the recipient's residual hearing and/or other cochlea health changes. For example, the cochlea health analysis module 118 may be configured to analyze ECAP and/or ECoG biomarkers in combination with a cochlea potential measurement, such as an intra-cochlea impedance measurement, to predict an upcoming/future residual hearing loss.

The electrophysiological signals measured within the cochlea relate to the operation of various biological functions of hearing, In contrast the cochlea potentials (electrical properties) as measured by the voltage telemetry of the implantable component relates to the measurement of the electrode interface and surrounding tissue electrical properties of the biological media surrounding the electrode array. As the electrophysiological signals such as the ECoG are indicative of cell function, access to information pertaining to biological content of the media surrounding the array may be important in the diagnosis of cochlea health. This information may be either employed as an independent measure or in combination with the electrophysiological data in an effort to either improve the accuracy of diagnosis or detection, or to improve the sensitivity of the system to detecting the onset of crises within the cochlea such that remedial actions may be taken for maximum clinical efficacy. For example, changes to the biological content of the perilymph surrounding the electrode array may result from an inflammatory response that may be detected prior to the manifestation of electrophysiological changes within the cochlea. In this example, the altered biological content may cause a change to the bulk impedance between electrode contacts. Similarly, the electrical properties of the cochlea (cochlea potentials) may be monitored to better describe or diagnose changes observed in the electrophysiological signal measured from the cochlea. Here, the changes in the electrophysiological signal, in this working example, the ECoG, can be caused by one of many changes in the inner ear. To best diagnose the root cause of the change (and best treatment), the electrical properties may be analyzed, in this example, including the electrode voltage spread within the cochlea to determine the nature of the change more specifically. An example being the development of hydrops whereby an increase in the pressure of the scala media may cause a bulging of the basilar membrane and alter the relative position of the electrode array in the scala tympani, the position of which can be determined from the change in the electrical properties.

In the example of FIG. 2, the analysis stage 252 may further comprise storing data relating to the classification of the biomarkers in a computer-readable storage medium 262. The computer-readable storage medium 262 may comprise one or more memories, and may be located on the electro-acoustic hearing prosthesis or remote from the electro-acoustic hearing prosthesis (e.g., the data may be stored via the Internet or the cloud).

As noted, the example of FIG. 2 illustrates that the techniques presented herein also utilize a remediation stage 254. The remediation stage 254 involves the initiation of one or more remedial actions in response to the identification of a residual hearing loss precursor (i.e., based on the analysis of the biomarkers during stage 252).

A number of different remedial actions may be initiated in accordance with the embodiments presented herein. For example, in certain embodiments a remedial action includes one or more adjustments to the operation of the electro-acoustic hearing prosthesis so as to proactively address the predicted changes in cochlea. That is, the results of the analysis stage 252 may be used to adjust one or more clinical parameters used by the electro-acoustic hearing prosthesis to generate the electro-acoustic signals in a manner that preserves the recipient's residual hearing (i.e., adjustments to electrical and/or acoustic clinical parameters). These adjustments can include adjustments configured to lessen the intensity and or overlap of the stimulation delivered to the cochlea (e.g., remove or minimize interactions between the electric and acoustic stimulation signals), prevent the incidence of excitotoxicity specific to cochlea sensory structures, minimize or mediate the onset of acoustic hearing loss (and in the event pharmacological agents are employed, optimizing the clinical parameters to complement drug therapy), or prevent the onset of neural fatigue or adaption. Adjustments to electrical clinical parameters to prevent cochlea health loss may also include adjustments to sequential clinical parameters (e.g. current level, pulse width, phase extender or third phase, rate or timing, mode, electrode, and frequency to electrode allocation), simultaneous clinical parameters (e.g., phased array parameters), and/or composite stimulation parameters. For example, the hearing prosthesis may reduce the instantaneous current level (CL) delivered to the cochlea by reducing the clinical current level on the electrode in question. In addition, as the charge of the stimulus is dictated by the current level and pulse width (PW), it may be possible to increase the pulse width and reduce the current level to maintain the same perceived loudness, but at the same time reduce the intensity of the stimulus as a remedial therapy to an identified cochlea health change. For stimulation overlap, the hearing prosthesis may either alter the frequency mapping of the acoustic and or electrical stimulus so that the stimulation is delivered to non-overlapping regions of the cochlea. This may be performed using imaging data of the electrode array insertion Adjustments to acoustic clinical parameters may include adjustments to, for example, applied frequencies (e.g., aidable frequencies and/or compressive frequency allocation), the crossover point/frequency (i.e., the frequency point or range indicating the areas/ranges that receive acoustic or electrical stimulation), sound intensity (e.g., gains, maximum power output, and/or maximum comfort level), and adaptive components (e.g., compression, such as onset, recovery, noise reduction, and/or directionality).

In summary of the above, the remedial actions initiated in response to the identification of a cochlea health change precursor are configured to counteract, terminate, or otherwise minimize the agitation/trigger of the cochlea health change.

The remediation stage 254 may also involve the administration of pharmacological agents in response to an inflammatory response and or dizziness post-operatively (i.e., drug therapy). For example, the electro-acoustic hearing prosthesis 100 may be configured to not only auto-regulate the clinical stimulus parameters (e.g., to determine if the symptoms abate), but also to administer regulated doses of pharmacological agents, such as corticosteroids. In certain circumstances, a remedial action can be the triggering of external alarms or delivery of communications to a caregiver via paired and/or connected communication devices.

In accordance with embodiments presented herein, once a remedial action is initiated, the cochlea health analysis module 118 is configured to monitor the efficacy of the remedial action. That is, the cochlea health analysis module 118 may continue to obtain and analyze one or more biomarkers, as described above, to ensure that the remedial action has achieved the underlying target effect (e.g., prevented the cochlea health change, slowed the cochlea health change, *etc.).* Again, this decision may be based on any one more of the biomarkers described above If the remedial action has not achieved the target effect (i.e., the biomarkers indicate a potential for a cochlea health change), the cochlea health analysis module 118 may initiate one or more additional remedial actions. In certain examples, the failure to achieve a target effect may cause a shut-down of the hearing prosthesis, cause the hearing prosthesis to enter a "safe" mode of operation where, for example, only minimal or critical stimulation is presented to the recipient, or may cause the deliver of an alarm and/or the transmission communication to a caregiver, clinician, *etc.*

As noted, in certain embodiments, the analysis of the biomarkers includes a classification of the type and/or cause of the residual hearing loss. This classification may be advantageous to determine, for example, the appropriate remedial action (e.g., clinical treatment) to initiate within remediation stage 254. For example, the classification may be used to determine if a remedial action should be initiated immediately by the hearing prosthesis itself (e.g., adjust stimulation parameters) or if a less response (e.g., notification or a clinician or caregiver) is sufficient.

Provided below are several examples illustrating how the techniques presented herein may be utilized to proactively identify and prevent cochlea health losses. One skilled in the art will recognize that these following examples are illustrative applications of the techniques described herein.

### Example A: Excitotoxicity

In this example, the aim is to prevent excessive acoustic or electric stimulus of the cochlear from causing chronic depolarization of cochlear hearing structures, which may lead to progressive swelling of the synaptic connections to the neural elements, causing permanent damage to the auditory information pathway (or synapse). Thus, in this example, there is relatively little damage to the acoustic hearing structures before the synapse (i.e., the hair cells). Rather, damage primarily occurs to the post-synaptic structures (i.e., the neural elements used for electrical stimulation).

In this specific example, the cochlear microphonic and the auditory nerve neurophonic may be measured in response to a pure tone input, and changes in the auditory nerve neurophonic and/or the cochlear microphonic may be analyzed. In one embodiment, a number of tones may be used, for example by stepping from low to high frequencies. During analysis stage 252, the electro-acoustic hearing prosthesis compares the present relative size difference of the cochlear microphonic and the auditory nerve neurophonic to peri-operative size differences and/or post-operative size differences that predate the application of the stimulation. If a change is detected in the shape and/or size of the response of the auditory nerve neurophonic, then the electro-acoustic hearing prosthesis provides a treatment by reducing the electro-acoustic stimulation over the course of hours until the relative size difference in the signals is reduced or close to zero.

### Example B: Conductive Hearing Loss

In this example, the aim is to predict the decrement in residual hearing that results from the growth of the fibrous tissue sheath around the intra-cochlear stimulating assembly post-implantation. In cases where the stimulating assembly has been inserted atraumatically and the tip or other parts of the array are resting in a position proximal, but not touching, the basilar membrane, there is a high probability that the growth of the tissue surrounding the stimulating assembly with touch or impinge on the basilar membrane. This physical presence can cause the residual hearing to degrade due to a change in the biophysical properties of the cochlear acoustic hearing. Contact with a biomechanical structure such as the basilar membrane can be detected by examining the resonant properties of the system (e.g., contact with the basilar membrane causes a stiffening of the system, resulting in a shift in the resonant properties). ECoG measurements can be analyzed at a number of frequencies to determine this resonant frequency shift. In extreme cases, ECoG measurements can also determine an impingement of the organ of corti.

In one specific example, the cochlear microphonic and the auditory nerve neurophonic are measured in response to a number of low and high frequency tones. An analysis involves comparing the present relative size difference the cochlear microphonic and the auditory nerve neurophonic to peri-operative size differences and/or post-operative size differences that predate the application of the stimulation, and calculating the cochlear microphonic/ auditory nerve neurophonic ratio. A determination can then be made as to whether or not there is a shift in the maximum response of the cochlear microphonic with respect to a baseline across a frequency spectrum. The analysis further comprises determining whether there is a change in the cochlear microphonic/Action Potential ratio with respect to a baseline across a frequency spectrum. If there is a change in the maximum response of the cochlear microphonic, the system may indicate a potential conductive hearing loss and signal for a re-mapping of the acoustic signals. If there is a change in the cochlear microphonic/Action Potential ratio, treatment is facilitated by flagging for impingement on a basilar membrane and signaling for a re-mapping of the electric signals.

### Example C: Neural Fatigue/Adaption

In this example, the aim is to predict and manage the clinical efficacy of the delivered electro-acoustic stimulation. Acoustic signals alone can cause the outer hair cells to adapt, which is characterized by a drop in the cochlear microphonic and/or auditory nerve neurophonic peak-to-peak amplitude at a given frequency on the order of 15-20 milliseconds (lower frequencies apply to electro-acoustic signals). Electric signals alone can cause fatigue or adaption at the neural level (i.e., an increase in the neural thresholds), which is characterized by a decrease in the ECAP on the order of milliseconds or seconds in response to chronic delivery of electrical stimulation. Combined electro-acoustic stimulation can cause outer hair cell and/or neural fatigue or adaption.

A measurement of the cochlear microphonic and/or auditory nerve neurophonic can be made by applying an acoustic tonal input at lower frequencies. Any changes to the cochlear microphonic amplitude over the course of milliseconds are monitored and recorded. An analysis determines whether the changes are beyond a threshold. If so, a remedial action is provided by decreasing the acoustic input (e.g., by adjusting compression or gains) and re-measuring and monitoring the cochlear microphonic amplitude. For cases involving electrical stimulation, an identical process may be followed by measuring ECAP response instead of cochlear microphonic amplitude.

### Example D: Immunological Response

In this example, the aim is to predict the onset of an immunological response within the cochlea before permanent damage can occur to the cochlea hearing structures. An immune response may occur in response to an acute infection within the cochlea; this is characterized by a change in the electrical properties of the cochlea (e.g., impedance).

A measurement of the cochlear microphonic and/or auditory nerve neurophonic can be made by applying an acoustic tonal input at lower frequencies. A further measurement can be made of the electrical properties, such as the impedance. Any changes to the cochlear microphonic amplitude and/or electrical properties over the course of hours are monitored and recorded. An analysis determines whether a change is detected in both the cochlear microphonic and electrical properties, whether the changes are permanent (i.e., do not restore after stimulation ceases or drops due to a quiet environment), and whether the changes are beyond a threshold. If so, the electro-acoustic hearing prosthesis applies a remedial action comprising decreasing the acoustic input (e.g., by adjusting compression or gains) and re-measuring and monitoring the cochlear microphonic, auditory nerve neurophonic, and/or electrical properties, flagging an alarm, and administering drug therapy if the implant is equipped with a built-in pump.

Examples A-D, above, provide specific embodiments whereby changes to stimulation parameters may be made such that further deterioration of residual hearing can be prevented.

In an embodiment, the electro-acoustic hearing prosthesis has access to the computer-readable storage medium described above. The computer-readable storage medium may store data relating to other recipients' biomarkers and classifications and treatments thereof. Applying statistics to the data allows for the electro-acoustic hearing prosthesis to apply the most statistically valid treatment, thereby ensuring maximal clinical utility and allowing the treatment to adapt as the data grows and evolves.

As noted, it is well-established that recipients of electro-acoustic hearing prostheses may present with mild to severe episodes of dizziness or vertigo following implantation. While the exact cause of dizziness or vertigo remains somewhat unclear in this context, it is thought that, in some cases, dizziness or vertigo is a secondary effect of an underlying disruption to increased pressure within the endolymphatic space or an acute or chronic inflammatory response within the inner ear. Thus, the example of FIG. 2 may indirectly treat dizziness or vertigo by reducing the odds of a recipient developing a perceivable residual hearing loss.

Developments in electro-acoustic hearing prosthesis technology provide for chronic delivery of electrical and/or acoustic stimulation. Chronic stimulation can exacerbate the above-described causes of perceivable residual hearing loss. The example of FIG. 2 combats the negative effects of chronic stimulation by mitigating the possible residual hearing loss.

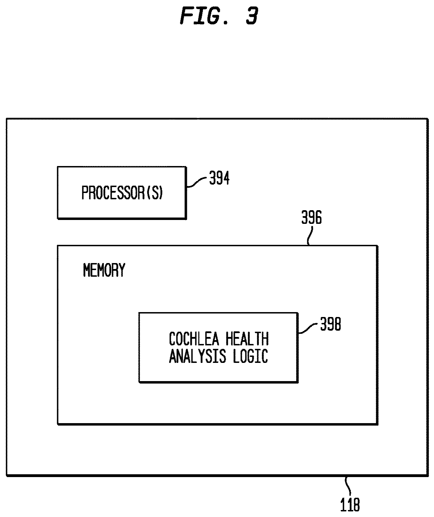

FIG. 3 is a schematic block diagram illustrating an arrangement for cochlea health analysis module 118 in accordance with an embodiment of the present invention. As shown, the hearing outcome tracking module 118 includes one or more processors 394 and a memory 396. The memory 396 includes cochlea health analysis logic 398.

The memory 396 may be read only memory (ROM), random access memory (RAM), or another type of physical/tangible memory storage device. Thus, in general, the memory 396 may comprise one or more tangible (non-transitory) computer readable storage media (e.g., a memory device) encoded with software comprising computer executable instructions and when the software is executed (by the one or more processors 394) it is operable to perform the operations described herein with reference to cochlea health analysis module 118. In one example, memory 396 includes the computer readable medium 262 described above with reference to FIG. 2.

FIG. 3 illustrates a specific software implementation for cochlea health analysis module 118. However, it is to be appreciated that cochlea health analysis module 118 may have other arrangements. For example, cochlea health analysis module 118 may be partially or fully implemented with digital logic gates in one or more application-specific integrated circuits (ASICs). Alternatively, the one or more processors 394 of hearing outcome tracking module may be the same or different processor as the sound processor 112 (FIGs. 1A-1C).

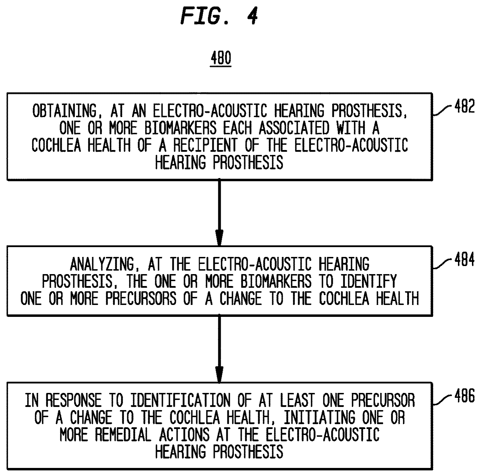

FIG. 4 is a flowchart illustrating an example method 480 in accordance with embodiments presented herein. Method 480 begins at 482 where an electro-acoustic hearing prosthesis obtains one or more biomarkers each associated with a cochlea health of a recipient of the electro-acoustic hearing prosthesis. At 484, the electro-acoustic hearing prosthesis analyzes, in real-time, the one or more biomarkers to identify one or more precursors of a change to the cochlea health. At 486, in response to identification of a precursor of a change to the cochlea health, the electro-acoustic hearing prosthesis initiates one or more remedial actions.

As described above, presented herein are techniques for monitoring biomarkers that relate to the cochlea health of a recipient of a hearing prosthesis in order to identify precursors to changes in the cochlea health. Given the transient and time dependent nature of the cochlea health changes, the hearing prosthesis may adapt one or more clinical parameters (e.g., acoustic and/or electrical stimulation parameters) to counter any observed changes considered to be deliterious to the residual hearing of the recipient.

It is to be appreciated that the embodiments presented herein are not mutually exclusive.

The description is not to be limited in scope by the specific preferred embodiments herein disclosed, since these embodiments are intended as illustrations, and not limitations. Indeed, various modifications in addition to those shown and described herein will become apparent to those skilled in the art from the foregoing description , the invention being defined by the appended claims.

## Claims

1. A hearing prosthesis (100), comprising:
a stimulating assembly (126) configured to be implanted in a recipient, wherein the stimulating assembly comprises a plurality of stimulating contacts (138) configured to deliver electrical stimulation signals to the recipient; and
a processor (112) configured to:
initiate at least one measurement via one or more of the stimulating contacts (138) to obtain one or more cochlea health biomarkers, each biomarker being associated with a cochlea health of the recipient of the hearing prosthesis;
analyze the one or more biomarkers to identify one or more precursors of a change to the residual hearing of the recipient to predict, using the one or more cochlea health biomarkers, a future change to residual hearing of the recipient's cochlea; and
responsive to the prediction of the future change to residual hearing , adjust stimulation signals of the hearing prosthesis (100) to proactively address the predicted future change to residual hearing.

2. The hearing prosthesis (100) of claim 1, wherein adjusting stimulation signals of the hearing prosthesis to proactively address the predicted future change to residual hearing includes adjusting stimulation signals in a manner that preserves the recipient's residual hearing.

3. The hearing prosthesis (100) of claim 1, wherein adjusting stimulation signals of the hearing prosthesis to proactively address the predicted future change to residual hearing includes adjustments configured to lessen the intensity of the stimulation delivered to the cochlea, and/or adjustments configured to alter the frequency mapping of the acoustic and/or electrical stimulus so that the stimulation is delivered to non-overlapping regions of the cochlea.

4. The hearing prosthesis (100) of claim 1, wherein adjusting stimulation signals of the hearing prosthesis to proactively address the predicted future change to residual hearing includes adjustments configured to increase a pulse width and reduce a current level.

5. The hearing prosthesis (100) of claim 1, wherein adjusting stimulation signals of the hearing prosthesis to proactively address the predicted future change to residual hearing includes adjustments configured to prevent, counteract, terminate, or otherwise minimize the predicted future change to residual hearing.

6. The hearing prosthesis (100) of any preceding claim, wherein the processor (112) is configured to:
initiate at least one electrocochleography measurement via one or more of the stimulating contacts (138) to obtain at least one of the one or more cochlea health biomarkers.

7. The hearing prosthesis (100) of any preceding claim, wherein the processor (112) is configured to:
initiate at least one electrically evoked compound action potential measurement via one or more of the stimulating contacts (138) to obtain at least one of the one or more cochlea health biomarkers.

8. The hearing prosthesis (100) of any preceding claim, wherein the processor (112) is configured to:
initiate at least one cochlea potential measurement via one or more of the stimulating contacts (138) to obtain at least one of the one or more cochlea health biomarkers.

9. The hearing prosthesis of any preceding claim, wherein the processor (100) is configured to:
classify future change to residual hearing by at least one of a type or cause of the future change to residual hearing.

10. The hearing prosthesis (100) of any preceding claim, wherein:
the one or more cochlea health biomarkers comprise a result of one or more cochlea potential measurements and a result of least one of an electrocochleography measurement and an electrically evoked compound action potential measurement.

11. The hearing prosthesis (100) of any preceding claim, wherein to predict residual hearing changes of the recipient's cochlea, the processor (112) is configured to:
identify electro-acoustic phenomena within the recipient's cochlea that are suggestive of residual hearing changes.

12. The hearing prosthesis (100) of any preceding claim, wherein the hearing prosthesis is an electro-acoustic hearing prosthesis including a receiver (142) configured to deliver acoustic stimulation signals to the recipient, and to adjust operation of the hearing prosthesis to remediate a future change to residual hearing , the processor (112) is configured to:
adjust one or more attributes of at least one of the electrical stimulation signals and the acoustic stimulation signals to substantially minimize interactions between the electrical and acoustic stimulation signals; and/or
adjust one or more attributes of at least one of the electrical stimulation signals and the acoustic stimulation signals to remediate the incidence of excitotoxicity specific to cochlea sensory structures; and/or
adjust one or more attributes of at least one of the electrical stimulation signals and the acoustic stimulation signals to substantially minimize onset of at least one of neural fatigue and adaption.

## Patentansprüche

1. Hörprothese (100), die Folgendes beinhaltet:
eine Stimulierungsanordnung (126), die zur Implantation in einen Empfänger konfiguriert ist, wobei die Stimulierungsanordnung mehrere Stimulierungskontakte (138) beinhaltet, die zum Liefern elektrischer Stimulationssignale an den Empfänger konfiguriert sind; und
einen Prozessor (394), der konfiguriert ist zum:
Einleiten wenigstens einer Messung über ein oder mehr Stimulierungskontakte (138) zum Ermitteln von ein oder mehr Cochlea-Gesundheits-Biomarkern, wobei jeder Biomarker mit einer Cochlea-Gesundheit des Empfängers der Hörprothese verbunden ist;
Analysieren der ein oder mehr Biomarker zum Identifizieren von ein oder mehr Vorläufern einer Änderung des Restgehörs des Empfängers zum Vorhersagen, unter Verwendung der ein oder mehr Cochlea-Gesundheits-Biomarker, einer zukünftigen Änderung des Restgehörs der Cochlea des Empfängers, und,
auf die Vorhersage der zukünftigen Änderung des Restgehörs reagierend, Einstellen von Stimulationssignalen der Hörprothese (100) zum proaktiven Angehen der vorhergesagten zukünftigen Änderung des Restgehörs.

2. Hörprothese (100) nach Anspruch 1, wobei das Einstellen von Stimulationssignalen der Hörprothese zum proaktiven Angehen der vorhergesagten zukünftigen Änderung des Restgehörs das Einstellen von Stimulationssignalen in einer Weise aufweist, die das Restgehör des Empfängers erhält.

3. Hörprothese (100) nach Anspruch 1, wobei das Einstellen von Stimulationssignalen der Hörprothese zum proaktiven Angehen der vorhergesagten zukünftigen Änderung des Restgehörs Einstellungen, die zum Verringern der Intensität der an die Cochlea übermittelten Stimulation konfiguriert sind, und/oder Einstellungen, die zum Verändern der Frequenzzuordnung des akustischen und/oder elektrischen Reizes konfiguriert sind, so dass die Stimulation an nicht-überlappende Regionen der Cochlea übermittelt wird, aufweist.

4. Hörprothese (100) nach Anspruch 1, wobei das Einstellen von Stimulationssignalen der Hörprothese zum proaktiven Angehen der vorhergesagten zukünftigen Änderung des Restgehörs Einstellungen aufweist, die zum Vergrößern der Pulsbreite und Verringern eines Stromniveaus konfiguriert sind.

5. Hörprothese (100) nach Anspruch 1, wobei das Einstellen von Stimulationssignalen der Hörprothese zum proaktiven Angehen der vorhergesagten zukünftigen Änderung des Restgehörs Einstellungen aufweist, die konfiguriert sind, um die bzw. der vorhergesagte(n) zukünftige(n) Änderung des Restgehörs zu verhindern, entgegenzuwirken, abzubrechen oder anderweitig zu minimieren.

6. Hörprothese (100) nach einem der vorhergehenden Ansprüche, wobei der Prozessor (112) konfiguriert ist zum:
Einleiten wenigstens einer Elektrocochleographiemessung über ein oder mehr der Stimulierungskontakte (138) zum Ermitteln von wenigstens einem der ein oder mehr Cochlea-Gesundheits-Biomarker.

7. Hörprothese (100) nach einem der vorhergehenden Ansprüche, wobei der Prozessor (112) konfiguriert ist zum:
Einleiten wenigstens einer Messung der elektrisch evozierten Summenaktionspotentiale über ein oder mehr der Stimulierungskontakte (138) zum Ermitteln von wenigstens einem der ein oder mehr Cochlea-Gesundheits-Biomarker.

8. Hörprothese (100) nach einem der vorhergehenden Ansprüche, wobei der Prozessor (112) konfiguriert ist zum:
Einleiten wenigstens einer Cochlea-Potentialmessung über ein oder mehr der Stimulierungskontakte (138) zum Ermitteln von wenigstens einem der ein oder mehr Cochlea-Gesundheits-Biomarker.

9. Hörprothese nach einem der vorhergehenden Ansprüche, wobei der Prozessor (112) konfiguriert ist zum:
Klassifizieren einer zukünftigen Änderung des Restgehörs nach wenigstens einem von einem Typ oder einer Ursache der zukünftigen Änderung des Restgehörs.

10. Hörprothese (100) nach einem der vorhergehenden Ansprüche, wobei:
die ein oder mehr Cochlea-Gesundheits-Biomarker ein Ergebnis von ein oder mehr Cochlea-Potentialmessungen und ein Ergebnis von wenigstens einem von einer Elektrocochleographiemessung und einer Messung der elektrisch evozierten Summenaktionspotentiale beinhalten.

11. Hörprothese (100) nach einem der vorhergehenden Ansprüche, wobei der Prozessor (112) zum Vorhersagen von Änderungen des Restgehörs der Cochlea des Empfängers konfiguriert ist zum:
Identifizieren von elektroakustischen Phänomenen innerhalb der Cochlea des Empfängers, die auf Änderungen des Restgehörs hindeuten.

12. Hörprothese (100) nach einem der vorhergehenden Ansprüche, wobei die Hörprothese eine elektroakustische Hörprothese ist, die einen Empfänger (142) aufweist, der zum Liefern akustischer Stimulationssignale an den Empfänger und zum Einstellen des Betriebs der Hörprothese zur Behebung einer zukünftigen Änderung des Restgehörs konfiguriert ist, wobei der Prozessor (112) konfiguriert ist zum:
Einstellen von ein oder mehr Attributen von wenigstens einem von den elektrischen Stimulationssignalen und den akustischen Stimulationssignalen, um Wechselwirkungen zwischen den elektrischen und akustischen Stimulationssignalen im Wesentlichen zu minimieren; und/oder
Einstellen von ein oder mehr Attributen von wenigstens einem von den elektrischen Stimulationssignalen und den akustischen Stimulationssignalen, um die Inzidenz von für sensorische Cochlea-Strukturen spezifische Exzitotoxizität zu beheben; und/oder
Einstellen von ein oder mehr Attributen von wenigstens einem von den elektrischen Stimulationssignalen und den akustischen Stimulationssignalen, um das Einsetzen von wenigstens einem von neuraler Erschöpfung und Adaption im Wesentlichen zu minimieren.

## Revendications

1. Prothèse auditive (100), comprenant :
un ensemble de stimulation (126) configuré pour être implanté chez un receveur, dans laquelle l'ensemble de stimulation comprend une pluralité de contacts de stimulation (138) configurés pour fournir des signaux de stimulation électrique au receveur ; et
un processeur (112) configuré pour :
instituer au moins une mesure via un ou plusieurs contacts de stimulation (138) pour obtenir un ou plusieurs biomarqueurs de santé cochléaire, chaque biomarqueur étant associé à la santé cochléaire du receveur de la prothèse auditive ;
analyser l'un ou les plusieurs biomarqueurs pour identifier un ou plusieurs précurseurs d'un changement dans la capacité auditive résiduelle du receveur afin de prédire, en utilisant un ou plusieurs biomarqueurs de santé cochléaire, un changement futur à la capacité auditive résiduelle de la cochlée du receveur ; et
en réponse à la prédiction du changement futur à la capacité auditive résiduelle, ajuster les signaux de stimulation de la prothèse auditive (100) afin de répondre de manière proactive au changement futur prédit à la capacité auditive résiduelle.

2. Prothèse auditive (100) selon la revendication 1, dans laquelle ajuster les signaux de stimulation de la prothèse auditive pour répondre de manière proactive au changement futur prédit à la capacité auditive résiduelle comprend ajuster les signaux de stimulation d'une manière qui préserve la capacité auditive résiduelle du receveur.

3. Prothèse auditive (100) selon la revendication 1, dans laquelle ajuster les signaux de stimulation de la prothèse auditive afin de répondre de manière proactive au changement futur prédit à la capacité auditive résiduelle comprend des ajustements configurés pour atténuer l'intensité de la stimulation apportée à la cochlée, et/ou des ajustements configurés pour modifier la cartographie fréquentielle du stimulus acoustique et/ou électrique de sorte que la stimulation est apportée à des régions de la cochlée qui ne se chevauchent pas.

4. Prothèse auditive (100) selon la revendication 1, dans laquelle ajuster les signaux de stimulation de la prothèse auditive afin de répondre de manière proactive au changement futur prédit à la capacité auditive résiduelle comprend des ajustements configurés pour augmenter une largeur d'impulsion et réduire un niveau de courant.

5. Prothèse auditive (100) selon la revendication 1, dans laquelle ajuster les signaux de stimulation de la prothèse auditive afin de répondre de manière proactive au changement futur prédit à la capacité auditive résiduelle comprend des ajustements configurés pour prévenir, contrecarrer, terminer ou minimiser autrement le changement futur prédit à la capacité auditive résiduelle.

6. Prothèse auditive (100) selon l'une quelconque des revendications précédentes, dans laquelle le processeur (112) est configuré pour :
instituer au moins une mesure d'électrocochléographie via un ou plusieurs contacts de stimulation (138) afin d'obtenir au moins l'un de l'un ou plusieurs biomarqueurs de santé cochléaire.

7. Prothèse auditive (100) selon l'une quelconque des revendications précédentes, dans laquelle le processeur (112) est configuré pour :
instituer au moins une mesure de potentiel d'action composée suscité électriquement via un ou plusieurs des contacts de stimulation (138) afin d'obtenir au moins l'un de l'un ou plusieurs biomarqueurs de santé cochléaire.

8. Prothèse auditive (100) selon l'une quelconque des revendications précédentes, dans laquelle le processeur (112) est configuré pour :
instituer au moins une mesure de potentiel cochléaire via un ou plusieurs des contacts de stimulation (138) afin d'obtenir au moins l'un de l'un ou plusieurs biomarqueurs de santé cochléaire.

9. Prothèse auditive selon l'une quelconque des revendications précédentes, dans laquelle le processeur (100) est configuré pour :
classifier un changement futur à la capacité auditive résiduelle par au moins l'un d'un type ou d'une cause de changement futur à la capacité auditive résiduelle.

10. Prothèse auditive (100) selon l'une quelconque des revendications précédentes, dans laquelle :
l'un ou plusieurs biomarqueurs de santé cochléaire comprend un résultat de l'une ou plusieurs mesures de potentiel cochléaire et un résultat d'au moins une mesure d'électrocochléographie et d'une mesure de potentiel d'action composée suscité électriquement.

11. Prothèse auditive (100) selon l'une quelconque des revendications précédentes, dans laquelle pour prédire des changements de capacité auditive résiduelle de la cochlée du receveur, le processeur (112) est configuré pour :
identifier des phénomènes électro-acoustiques dans la cochlée du receveur qui suggèrent des changements de capacité auditive résiduelle.

12. Prothèse auditive (100) selon l'une quelconque des revendications précédentes, dans laquelle la prothèse auditive est une prothèse auditive électro-acoustique comprenant un récepteur (142) configuré pour fournir des signaux de stimulation acoustique au receveur, et pour ajuster le fonctionnement de la prothèse auditive afin de remédier à un changement futur à la capacité auditive résiduelle, le processeur (112) est configuré pour :
ajuster un ou plusieurs attributs d'au moins l'un des signaux de stimulation électrique et des signaux de stimulation acoustique afin de minimiser sensiblement des interactions entre les signaux de stimulation électrique et acoustique ; et/ou
ajuster un ou plusieurs attributs d'au moins l'un des signaux de stimulation électrique et des signaux de stimulation acoustique afin de remédier à l'incidence d'excitotoxicité spécifique aux structures sensorielles de la cochlée ; et/ou
ajuster un ou plusieurs attributs d'au moins l'un des signaux de stimulation électrique et des signaux de stimulation acoustique afin de minimiser sensiblement la survenue d'au moins l'une d'entre une fatigue neuronale et une adaptation.
